# EUROPEAN PATENT SPECIFICATION

(11) **EP 4 259 040 B1**
(45) Date of publication and mention of the grant of the patent: **19.02.2025**
(21) Application number: 21824569.4
(22) Date of filing: 06.12.2021
(51) Int. Cl.: A61F 2/24, D01D 5/00

(54) **REINFORCED HEART VALVE LEAFLETS**
VERSTÄRKTE HERZKLAPPENBLÄTTCHEN
FEUILLETS DE VALVULE CARDIAQUE RENFORCÉS

(30) Priority: 11.12.2020 US 202063124289 P
(43) Date of publication of application: 18.10.2023
(73) Proprietor: Xeltis, AG, 8008 Zürich (CH)
(72) Inventor: LALIEU, Roland Lambertus Joannes, 6006 CR Weert (NL); COX, Martijn Antonius Johannes, 6021 RC Budel (NL)
(74) Representative: FRKelly
(86) International application number: PCT/EP2021/084381
(87) International publication number: WO 2022/122647

(56) References cited:
- WO-A2-03/099230
- US-A1- 2014 207 250
- US-A1- 2019 201 588
- US-A1- 2019 271 098

## Description

### FIELD OF THE INVENTION

This invention relates to restorative heart valves.

### BACKGROUND OF THE INVENTION

A natural heart valve has a highly-organized tri-layer structure capable of coping with forces placed upon it while being thin and flexible enough to open and close properly. It has three distinct layers, each layer with unique structural and functional properties, and a preferred alignment of tissue fibers beneficial for the performance of the heart valve.

Single layered prosthetic heart valves or restorative heart valves which replace natural heart valves are normally made from pericardium and have limited long term durability. However, artificial scaffolds for heart valves do not adequately mimic the mechanical properties of natural donor scaffolds. During durability tests of the current valve design leaflets tend to rupture, mainly in radial direction. Accordingly, there is a need for a new scaffold material that has a higher durability while still maintaining the desired and needed mobility. The new scaffold should also have aligned reinforcement with different strength in different areas / directions. The present invention provides an electrospinning method specifically designed for heart valves to address at least some of the problems.

US 2014/207250 A1 relates to the process of forming a tissue scaffold, the process comprising electrospinning polymer fibers from a spinneret onto a tissue scaffold template. In embodiments, the fibers are plasma-treated between the spinneret and the template. The scaffold may be constructed of alternating layers of aligned and randomly oriented fibers. The scaffold may be heat treated to control the mechanical properties of the scaffold, such as density, pore size and pore interconnectivity.

US 2019/271098A1 is directed to flexible electrospun fiber rods and methods of manufacturing such rods. A scaffold may comprise a flexible rod having a spiral cross-section, the flexible rod comprising electrospun polymer fibers. The electrospun polymer fibers may be substantially aligned or randomly oriented with respect to one another. The flexible rod may further comprise substantially uniformly distributed pores. The flexible rod may have a length and a diameter of a native mammalian tendon or ligament. A method of manufacturing such a scaffold may comprise forming a layer of polymer fibers on a mandrel by electrospinning, rolling the layer from a first end of the mandrel to a second end of the mandrel to form a toroid at the second end of the mandrel, and cutting the toroid off the mandrel to form a flexible rod having a spiral cross-section.

WO 03/099230 A2 is directed to formation and use of electroprocessed collagen, including use as an extracellular matrix and, together with cells, its use in forming engineered tissue. The engineered tissue can include the synthetic manufacture of specific organs or tissues which may be implanted into a recipient. The electroprocessed collagen may also be combined with other molecules in order to deliver substances to the site of application or implantation of the electroprocessed collagen. The collagen or collagen/cell suspension is electrodeposited onto a substrate to form tissues and organs.

US 2019/201588 A1 relates to a biodegradable cardiovascular implant for growing cardiovascular tissue in a patient. The implant distinguishes an electro-spun network with supramolecular compounds having hard-blocks covalently bonded with soft-blocks resulting in much enhanced durability and fatigue resistance, while maintaining the effectiveness as a cardiovascular implant.

### SUMMARY OF THE INVENTION

In one embodiment the invention is a method of electrospinning a heart valve leaflet. Aligned fibers are electrospun onto a mandrel using a first source. The aligned fibers are bioabsorbable polymer fibers. Non-aligned fibers are electrospun onto the mandrel using a second source. The second source electospins simultaneously with the first source and, these non-aligned fibers are bioabsorbable polymer fibers. The electrospinning of the aligned fibers includes electrospinning distinct regions at the mandrel therewith producing a circumferentially aligned band of the heart valve leaflet. The aligned fibers and the non-aligned fibers at the distinct regions are electrospun as an intermixed pattern. The circumferentially aligned band can be from commissure to commissure at or near the free edge of the heart valve leaflet, or the circumferentially aligned band is used to reinforce a belly area of the heart valve leaflet.

In another embodiment, the invention pertains to a heart valve with at least one leaflet. A free edge of the leaflet comprises or consists essentially of a circumferentially aligned band of aligned electrospun fibers with non-aligned fibers. The aligned fibers are bioabsorbable polymer fibers, the non-aligned fibers are bioabsorbable polymer fibers, and the aligned fibers and the non-aligned fibers at the free edge are intermixed.

In still another embodiment, the invention pertains to a heart valve with at least one leaflet. The leaflet belly area comprises or consists essentially of a circumferentially aligned band of aligned electrospun fibers with non-aligned fibers. The aligned fibers are bioabsorbable polymer fibers, the non-aligned fibers are bioabsorbable polymer fibers, and the aligned fibers and the non-aligned fibers at the free edge are intermixed.

In the embodiments, the bioabsorbable polymer fibers of the aligned fibers can be supramolecular bioabsorbable polymer fibers and/or the bioabsorbable polymer fibers of the non-aligned fibers can be supramolecular bioabsorbable polymer fibers.

In the embodiments, the bioabsorbable polymer fibers of the aligned fibers form a porous network to allow infiltration of cells, and the porous network with infiltrated cells is capable of being replaced over time with newly formed tissue, and/or the bioabsorbable polymer fibers of the non-aligned fibers form a porous network to allow infiltration of cells, and the porous network with infiltrated cells is capable of being replaced over time with newly formed tissue.

### BRIEF DESCRIPTION OF THE DRAWINGS

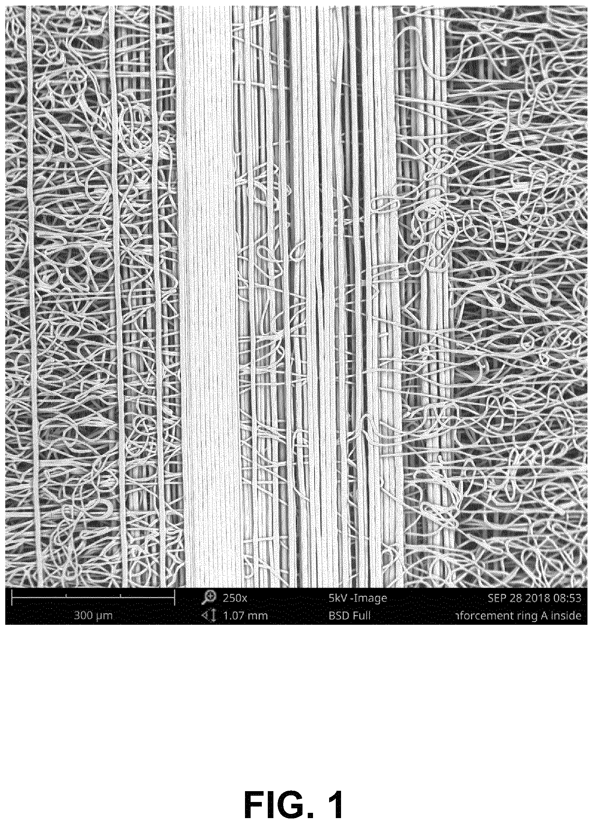

- **FIG. 1**: shows according to an exemplary embodiment of the invention an enforcement ring of aligned fibers (view from outside), spun simultaneously with random fibers, yielding an intertwined pattern.

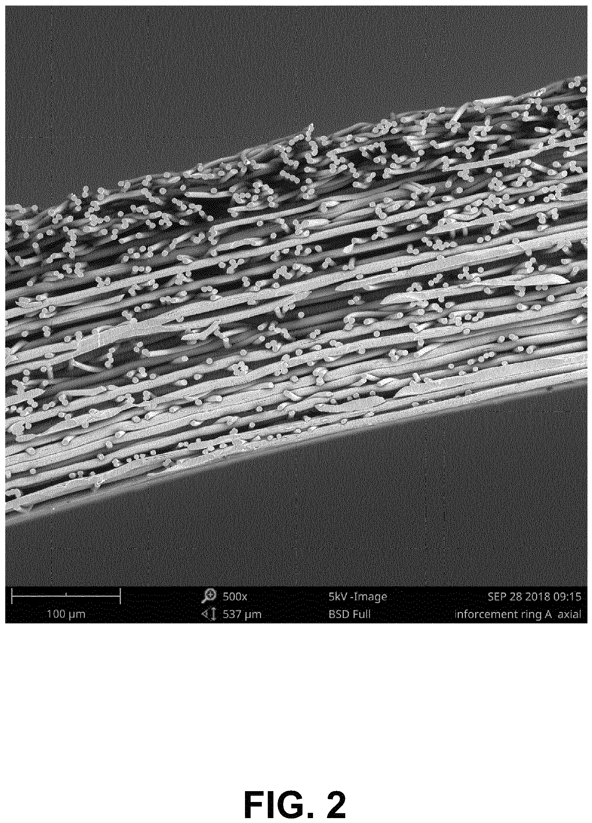

- **FIG. 2**: shows according to an exemplary embodiment of a matrix of aligned fibers alternating with random fibers (sectional view).
- **FIG. 3**: shows according to an exemplary embodiment of the invention a method of electrospinning using two sources simultaneously with one source for aligned fibers and the other source for non-aligned fibers to form a heart valve.

### DETAILED DESCRIPTION

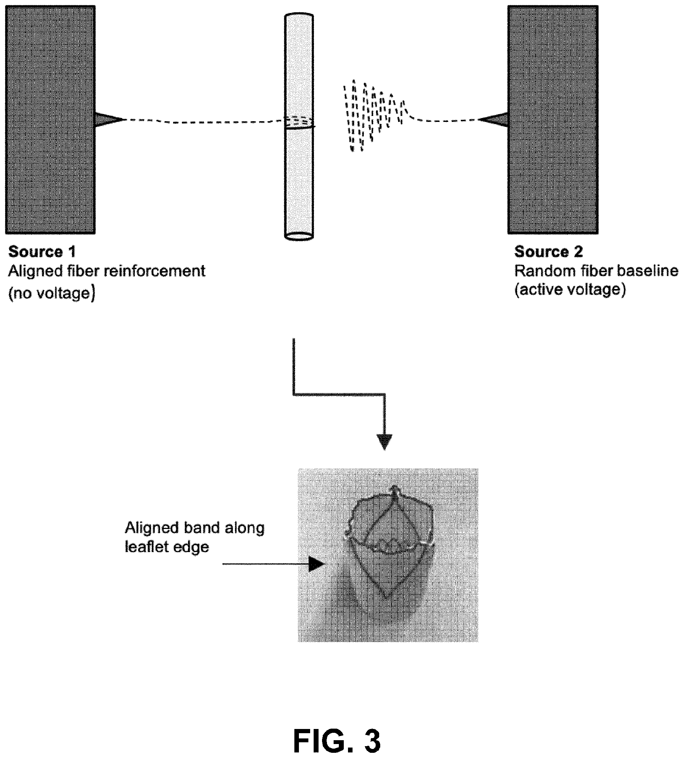

Embodiments of the invention pertain to a method and a method of making reinforced heart valve leaflets. In one example, the invention is a method of:
- Simultaneous spinning of aligned and non-aligned fibers to form a heart valve that is capable of allowing endogenous tissue restoration (ETR). Simultaneous spinning is capable of creating a microstructure that allows ETR and improves integration and adhesion.
- Simultaneous electrospinning using two sources, where one source produces a straight jet and the other source produces random fibers (FIG. 3). The advantage of the straight jet is a more precise control of location of deposition. The advantage of simultaneous electrospinning using two sources is that this ensures (i) better adhesion between aligned and random structures, and (ii) improves porosity by avoiding too dense aligned regions. A straight jet could alternatively be achieved through a kind of melt extrusion process (e.g. electrospinning without voltage difference).
- Creating specific locations of fiber reinforcement of the heart valve, which is achievable due to the precise control of the straight jet. For example, a circumferentially aligned band along the free edge of the heart valve, going from commissure to commissure. In another example, a curvilinear circumferentially aligned set of bands/bundles, running from commissure to commissure. In yet another example, a circumferentially aligned band/bundle in the belly area of a leaflet.
- In one embodiment, the use of supramolecular polymers for this method.

The artificially engineered electrospun heart valve has at least a layer of aligned fibers and at least a layer of randomly oriented fibers. With this approach, it is possible to then generate a heart valve having mechanical properties which more closely match those of a natural donor scaffold with regard to stiffness and strength.

In one embodiment, very directed, aligned fibers are produced, which are intermixed with more randomly oriented "normal" electrospun fibers without creating distinct layers. This leads to a heart valve tissue with aligned and non-aligned fibers that are in direct connection to each other and interwoven.

In another configuration not covered by the claims, aligned fibers are produced in distinct regions to enforce the resulting heart valve tissue. This results in distinct regions of aligned fibers that may be alternating with non-aligned fibers.

In yet another embodiment, the aligned fibers are produced simultaneously with non-aligned fibers, thus creating stronger interaction and adhesion between aligned and non-aligned fibers, which is beneficial for preventing delamination, and may provide a more optimal distribution of local porosity (for cell ingrowth and ETR).

In yet another embodiment, the aligned fibers are produced as a straight jet, thus allowing more controlled deposition.

In yet another embodiment, the straight jet to produce aligned fibers is produced through a melt-extrusion process or through an electrospinning process or through a winding process.

In yet another embodiment, the aligned fibers form a circumferentially aligned band along the free edge of a heart valve leaflet, going from commissure to commissure.

In yet another embodiment, the aligned fibers form a curvilinear circumferentially aligned set of bands/bundles, running from commissure to commissure.

In yet another embodiment, the aligned fibers form a circumferentially aligned band/bundle in the belly area of a leaflet.

In yet another embodiment, the aligned fibers would be deposited on a leaflet in bands or bundles to optimize balance between leaflet durability and flexibility.

In yet another embodiment, the aligned fibers could have either an absorbable or a non-absorbable material.

In still another embodiment, the aligned fibers could be a suture wire for example made from UltraHigh Molecular Weight PolyEthylene (UHMWPE).

By spinning fibers in a more circumferential direction, these heart valves increase their durability.

The aligned fibers could be locally defined or dispersed. Alignment could be done in a preferred direction e.g. reinforcement into the circumferential direction could be preferred since forces onto the leaflet in the circumferential direction are bigger and could therefore lead to failure of the leaflet. The area of aligned fibers could be locally defined or dispersed over the whole area, aligned in a preferred direction and/or dispersed as regions or along the whole scaffold. By this approach leaflets could be reinforced and are more durable as a result. They could be reinforced by aligned fibers in different ways such as (bio-absorbable) wire, locally aligned fibers or a band.

Aligned fibers could be different in diameter, different in material or other characteristics. If needed, the aligned fiber polymer could be different from the non-aligned fiber polymer. The aligned fiber polymer could be more or less bio-absorbable, dependent on the needs.

By using a bio-absorbable wire the circumferential strength at specific areas is enhanced. These wires will be absorbed over-time like the rest of the heart valve, but will give support during first ETR to prevent tears. An aligned wire could be sutured through the coaptation plane of the leaflets or attached in other ways. An aligned circumferential band which supports the leaflets will prevent tearing as well. Alternatively, an aligned edge or rim could be added during production or after production onto or into the scaffold, e.g. by directly electrospinning on the scaffold.

In one example, the aligned and non-aligned fibers could be woven and/or interconnected.

The method as shown in FIG. 3 has a second polymer source that is used to apply aligned fibers during the production in addition to non-aligned fibers produced by a first polymer source. These aligned fibers can be either electrospun using a separate power supply or pulled from a continuous droplet supplied by a pump. This results in a very accurate positioning in the deposition area. Using this method, one could spin specific orientations on complex shaped targets for making heart valves.

The electrospun material referenced in this document may comprise the ureido-pyrimidinone (UPy) quadruple hydrogen-bonding motif (pioneered by Sijbesma (1997), Science 278, 1601-1604) and a polymer backbone, for example selected from the group of biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

The same result may be obtained with alternative, non-supramolecular polymers, if properties are carefully selected and material processed to ensure required surface characteristics. These polymers may comprise biodegradable or non-biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

## Claims

1. A method of electrospinning a heart valve leaflet, comprising:
(a) electrospinning aligned fibers onto a mandrel using a first source, wherein the aligned fibers are bioabsorbable polymer fibers; and
(b) electrospinning non-aligned fibers onto the mandrel using a second source, wherein the second source electospins simultaneously with the first source and, wherein the non-aligned fibers are bioabsorbable polymer fibers,
wherein the electrospinning of the aligned fibers includes electrospinning distinct regions at the mandrel therewith producing a circumferentially aligned band of the heart valve leaflet, and wherein the aligned fibers and the non-aligned fibers at the distinct regions are electrospun as an intermixed pattern.

2. The method as set forth in claim 1, wherein the bioabsorbable polymer fibers of the aligned fibers are supramolecular bioabsorbable polymer fibers and/or wherein the bioabsorbable polymer fibers of the non-aligned fibers are supramolecular bioabsorbable polymer fibers.

3. The method as set forth in claim 1, wherein the circumferentially aligned band is from commissure to commissure at or near the free edge of the heart valve leaflet, or wherein the circumferentially aligned band is used to reinforce a belly area of the heart valve leaflet.

4. The method as set forth in claim 1, wherein the bioabsorbable polymer fibers of the aligned fibers form a porous network to allow infiltration of cells, and wherein the porous network with infiltrated cells is capable of being replaced over time with newly formed tissue,
and/or wherein the bioabsorbable polymer fibers of the non-aligned fibers form a porous network to allow infiltration of cells, and wherein the porous network with infiltrated cells is capable of being replaced over time with newly formed tissue.

5. A heart valve, comprising at least one leaflet, wherein a free edge of the leaflet consists essentially of a circumferentially aligned band of aligned electrospun fibers with non-aligned fibers, wherein the aligned fibers are bioabsorbable polymer fibers, wherein the non-aligned fibers are bioabsorbable polymer fibers, and wherein the aligned fibers and the non-aligned fibers at the free edge are intermixed.

6. The heart valve as set forth in claim 5, wherein the bioabsorbable polymer fibers of the aligned fibers are supramolecular bioabsorbable polymer fibers and/or wherein the bioabsorbable polymer fibers of the non-aligned fibers are supramolecular bioabsorbable polymer fibers.

7. The heart valve as set forth in claim 5, wherein the bioabsorbable polymer fibers of the aligned fibers form a porous network to allow infiltration of cells, and wherein the porous network with infiltrated cells is capable of being replaced over time with newly formed tissue,
and/or wherein the bioabsorbable polymer fibers of the non-aligned fibers form a porous network to allow infiltration of cells, and wherein the porous network with infiltrated cells is capable of being replaced over time with newly formed tissue.

8. A heart valve, comprising at least one leaflet, wherein the leaflet belly area consists essentially of a circumferentially aligned band of aligned electrospun fibers with non-aligned fibers, wherein the aligned fibers are bioabsorbable polymer fibers, wherein the non-aligned fibers are bioabsorbable polymer fibers, and wherein the aligned fibers and the non-aligned fibers at the free edge are intermixed.

9. The heart valve as set forth in claim 8, wherein the bioabsorbable polymer fibers of the aligned fibers are supramolecular bioabsorbable polymer fibers and/or wherein the bioabsorbable polymer fibers of the non-aligned fibers are supramolecular bioabsorbable polymer fibers.

10. The heart valve as set forth in claim 8, wherein the bioabsorbable polymer fibers of the aligned fibers form a porous network to allow infiltration of cells, and wherein the porous network with infiltrated cells is capable of being replaced over time with newly formed tissue,
and/or wherein the bioabsorbable polymer fibers of the non-aligned fibers form a porous network to allow infiltration of cells, and wherein the porous network with infiltrated cells is capable of being replaced over time with newly formed tissue.

## Patentansprüche

1. Verfahren zum Elektrospinnen eines Herzklappenblättchens, Folgendes umfassend:
(a) Elektrospinnen von ausgerichteten Fasern auf einen Dorn unter Verwendung einer ersten Quelle, wobei die ausgerichteten Fasern bioresorbierbare Polymerfasern sind; und
(b) Elektrospinnen von nicht ausgerichteten Fasern auf den Dorn unter Verwendung einer zweiten Quelle, wobei die zweite Quelle gleichzeitig mit der ersten Quelle elektrospinnt und wobei die nicht ausgerichteten Fasern bioresorbierbare Polymerfasern sind,
wobei das Elektrospinnen der ausgerichteten Fasern Elektrospinnen ausgeprägter Bereiche an dem Dorn umfasst, wodurch ein umfänglich ausgerichtetes Band des Herzklappenblättchens erzeugt wird, und wobei die ausgerichteten Fasern und die nicht ausgerichteten Fasern an den ausgeprägten Bereichen als vermischtes Muster elektrogesponnen werden.

2. Verfahren nach Anspruch 1, wobei die bioresorbierbaren Polymerfasern der ausgerichteten Fasern supramolekulare bioresorbierbare Polymerfasern sind und/oder wobei die bioresorbierbaren Polymerfasern der nicht ausgerichteten Fasern supramolekulare bioresorbierbare Polymerfasern sind.

3. Verfahren nach Anspruch 1, wobei das umfänglich ausgerichtete Band von Kommissur zu Kommissur an oder nahe dem freien Rand des Herzklappenblättchens angeordnet ist, oder wobei das umfänglich ausgerichtete Band zur Verstärkung eines Bauchbereichs des Herzklappenblättchens verwendet wird.

4. Verfahren nach Anspruch 1, wobei die bioresorbierbaren Polymerfasern der ausgerichteten Fasern ein poröses Netzwerk bilden, um Infiltration von Zellen zu ermöglichen, und wobei das poröse Netzwerk mit infiltrierten Zellen im Laufe der Zeit durch neu gebildetes Gewebe ersetzt werden kann,
und/oder wobei die bioresorbierbaren Polymerfasern der nicht ausgerichteten Fasern ein poröses Netzwerk bilden, um Infiltration von Zellen zu ermöglichen, und wobei das poröse Netzwerk mit infiltrierten Zellen im Laufe der Zeit durch neu gebildetes Gewebe ersetzt werden kann.

5. Herzklappe, umfassend mindestens ein Klappenblättchen, wobei ein freier Rand des Klappenblättchens im Wesentlichen aus einem in Umfangsrichtung ausgerichteten Band aus ausgerichteten elektrogesponnenen Fasern mit nicht ausgerichteten Fasern besteht, wobei die ausgerichteten Fasern bioresorbierbare Polymerfasern sind, wobei die nicht ausgerichteten Fasern bioresorbierbare Polymerfasern sind, und wobei die ausgerichteten Fasern und die nicht ausgerichteten Fasern an dem freien Rand miteinander vermischt sind.

6. Herzklappe nach Anspruch 5, wobei die bioresorbierbaren Polymerfasern der ausgerichteten Fasern supramolekulare bioresorbierbare Polymerfasern sind und/oder wobei die bioresorbierbaren Polymerfasern der nicht ausgerichteten Fasern supramolekulare bioresorbierbare Polymerfasern sind.

7. Herzklappe nach Anspruch 5, wobei die bioresorbierbaren Polymerfasern der ausgerichteten Fasern ein poröses Netzwerk bilden, um Infiltration von Zellen zu ermöglichen, und wobei das poröse Netzwerk mit infiltrierten Zellen im Laufe der Zeit durch neu gebildetes Gewebe ersetzt werden kann,
und/oder wobei die bioresorbierbaren Polymerfasern der nicht ausgerichteten Fasern ein poröses Netzwerk bilden, um Infiltration von Zellen zu ermöglichen, und wobei das poröse Netzwerk mit infiltrierten Zellen im Laufe der Zeit durch neu gebildetes Gewebe ersetzt werden kann.

8. Herzklappe, umfassend mindestens ein Klappenblättchen, wobei der Bauchbereich des Klappenblättchens im Wesentlichen aus einem in Umfangsrichtung ausgerichteten Band aus ausgerichteten elektrogesponnenen Fasern mit nicht ausgerichteten Fasern besteht, wobei die ausgerichteten Fasern bioresorbierbare Polymerfasern sind, wobei die nicht ausgerichteten Fasern bioresorbierbare Polymerfasern sind, und wobei die ausgerichteten Fasern und die nicht ausgerichteten Fasern an dem freien Rand miteinander vermischt sind.

9. Herzklappe nach Anspruch 8, wobei die bioresorbierbaren Polymerfasern der ausgerichteten Fasern supramolekulare bioresorbierbare Polymerfasern sind und/oder wobei die bioresorbierbaren Polymerfasern der nicht ausgerichteten Fasern supramolekulare bioresorbierbare Polymerfasern sind.

10. Herzklappe nach Anspruch 8, wobei die bioresorbierbaren Polymerfasern der ausgerichteten Fasern ein poröses Netzwerk bilden, um Infiltration von Zellen zu ermöglichen, und wobei das poröse Netzwerk mit infiltrierten Zellen im Laufe der Zeit durch neu gebildetes Gewebe ersetzt werden kann,
und/oder wobei die bioresorbierbaren Polymerfasern der nicht ausgerichteten Fasern ein poröses Netzwerk bilden, um Infiltration von Zellen zu ermöglichen, und wobei das poröse Netzwerk mit infiltrierten Zellen im Laufe der Zeit durch neu gebildetes Gewebe ersetzt werden kann.

## Revendications

1. Procédé d'électrofilage d'un feuillet de valvule cardiaque comprenant :
(a) l'électrofilage de fibres alignées sur un mandrin par l'utilisation d'une première source, les fibres alignées étant des fibres polymères bioabsorbables ; et
(b) l'électrofilage de fibres non alignées sur le mandrin par l'utilisation d'une seconde source, la seconde source électrofilant simultanément avec la première source et les fibres non alignées étant des fibres polymères bioabsorbables,
dans lequel l'électrofilage des fibres alignées comporte l'électrofilage de régions distinctes au niveau du mandrin à l'aide ce dernier, produisant ainsi une bande alignée circonférentiellement du feuillet de la valvule cardiaque, et dans lequel les fibres alignées et les fibres non alignées au niveau des régions distinctes sont électrofilées sous la forme d'un motif entremêlé.

2. Procédé selon la revendication 1, dans lequel les fibres polymères bioabsorbables des fibres alignées sont des fibres polymères bioabsorbables supramoléculaires et/ou dans lequel les fibres polymères bioabsorbables des fibres non alignées sont des fibres polymères bioabsorbables supramoléculaires.

3. Procédé selon la revendication 1, dans lequel la bande alignée circonférentiellement s'étend d'une commissure à l'autre au niveau ou à proximité du bord libre du feuillet de la valvule cardiaque, ou dans lequel la bande alignée circonférentiellement est utilisée pour renforcer une zone ventrale du feuillet de la valvule cardiaque.

4. Procédé selon la revendication 1, dans lequel les fibres polymères bioabsorbables des fibres alignées forment un réseau poreux pour permettre l'infiltration de cellules, et dans lequel le réseau poreux avec des cellules infiltrées est susceptible d'être remplacé au fil du temps par du tissu nouvellement formé,
et/ou dans lequel les fibres polymères bioabsorbables des fibres non alignées forment un réseau poreux pour permettre l'infiltration de cellules, et dans lequel le réseau poreux avec des cellules infiltrées est susceptible d'être remplacé au fil du temps par du tissu nouvellement formé.

5. Valvule cardiaque comprenant au moins un feuillet, dans laquelle un bord libre du feuillet est constitué essentiellement d'une bande alignée circonférentiellement de fibres électrofilées alignées avec des fibres non alignées, dans laquelle les fibres alignées sont des fibres polymères bioabsorbables, dans laquelle les fibres non alignées sont des fibres polymères bioabsorbables, et dans laquelle les fibres alignées et les fibres non alignées au bord libre sont entremêlées.

6. Valvule cardiaque selon la revendication 5, dans laquelle les fibres polymères bioabsorbables des fibres alignées sont des fibres polymères bioabsorbables supramoléculaires et/ou dans laquelle les fibres polymères bioabsorbables des fibres non alignées sont des fibres polymères bioabsorbables supramoléculaires.

7. Valvule cardiaque selon la revendication 5, dans laquelle les fibres polymères bioabsorbables des fibres alignées forment un réseau poreux pour permettre l'infiltration de cellules, et dans laquelle le réseau poreux avec des cellules infiltrées est susceptible d'être remplacé au fil du temps par du tissu nouvellement formé,
et/ou dans laquelle les fibres polymères bioabsorbables des fibres non alignées forment un réseau poreux pour permettre l'infiltration de cellules, et dans laquelle le réseau poreux avec des cellules infiltrées est susceptible d'être remplacé au fil du temps par du tissu nouvellement formé.

8. Valvule cardiaque, comprenant au moins un feuillet, dans laquelle la zone ventrale du feuillet est constituée essentiellement d'une bande alignée circonférentiellement de fibres électrofilées alignées avec des fibres non alignées, dans laquelle les fibres alignées sont des fibres polymères bioabsorbables, dans laquelle les fibres non alignées sont des fibres polymères bioabsorbables, et dans laquelle les fibres alignées et les fibres non alignées au bord libre sont entremêlées.

9. Valvule cardiaque selon la revendication 8, dans laquelle les fibres polymères bioabsorbables des fibres alignées sont des fibres polymères bioabsorbables supramoléculaires et/ou dans laquelle les fibres polymères bioabsorbables des fibres non alignées sont des fibres polymères bioabsorbables supramoléculaires.

10. Valvule cardiaque selon la revendication 8, dans laquelle les fibres polymères bioabsorbables des fibres alignées forment un réseau poreux pour permettre l'infiltration de cellules, et dans laquelle le réseau poreux avec des cellules infiltrées est susceptible d'être remplacé au fil du temps par du tissu nouvellement formé,
et/ou dans laquelle les fibres polymères bioabsorbables des fibres non alignées forment un réseau poreux pour permettre l'infiltration de cellules, et dans laquelle le réseau poreux avec des cellules infiltrées est susceptible d'être remplacé au fil du temps par du tissu nouvellement formé.
